# EUROPEAN PATENT APPLICATION

(11) **EP 4 354 452 A1**
(43) Date of publication of application: **17.04.2024**
(21) Application number: 22200810.4
(22) Date of filing: 11.10.2022
(51) Int. Cl.: G16H 30/20, G16H 30/40, G06N 3/02

(54) **MEDICAL IMAGE SEARCH AND RETRIEVAL**

(71) Applicant: Koninklijke Philips N.V., 5656 AG Eindhoven (NL)
(72) Inventor: ANAND, Shreya, Eindhoven (NL); CHAKRABARTI, Biswaroop, Eindhoven (NL); JELFS, Sam Martin, 5656AG Eindhoven (NL); MENDOZA, Jose Luis Diaz, 5656AG Eindhoven (NL); VAN EE, Raymond, Eindhoven (NL)
(74) Representative: Philips Intellectual Property & Standards

(57) **Abstract**

A method for retrieving relevant images from an image database based on converting linguistic search criteria into image-based search criteria. User inputs are used to construct a base image from a combination of pre-formed graphical or visual image elements, and an image database is then queried with the base image using image-based searching. The resulting retrieved images may be exported to a user training system for use in training a user using the images.

## Description

### FIELD OF THE INVENTION

The present invention relates to a method for optimized search of medical images having defined anatomical requirements.

### BACKGROUND OF THE INVENTION

Advancement of technology, particularly with regards to data storage, has resulted in availability of a large amount of medical image data stored in institutional and pan-institutional PACS systems. This information has enormous potential value, in particular for use in training clinicians in diagnostic analysis, and also potentially in training AI algorithms for various diagnostic and other classification functions.

However, despite the availability of the image data, there is a challenge in sorting the image data to find and retrieve images and image sets based on defined query criteria. The standard way to search a PACS database is to search by keyword and rely on metadata tags of the images in order to identify relevant images that meet the search criteria. However, tags may not always be reliable, or may not be as comprehensive as they could be. Some images may have no descriptive tags at all.

Therefore, there is a need for an improved means of obtaining image data sets from image databases based on defined criteria.

### SUMMARY OF THE INVENTION

The invention is defined by the claims.

According to examples in accordance with an aspect of the invention, there is provided a computer-implemented method comprising: selecting a plurality of pre-formed image elements from a pre-defined set of pre-formed image elements, each image element being an image representation of an anatomical object or feature; defining a relative positioning and/or size of the selected one or more image elements, wherein the selection of the plurality of image elements from the set, and the defining of the positioning and/or size of the image elements is performed based on user inputs received from a user interface device; constructing an artificial base image based on combining the selected image elements in accordance with the defined positioning and/or size; accessing an image database storing a dataset of medical images; performing an image-based searching operation comprising searching the image database based on the base image for finding images in the database similar to the base image; and generating a data package for export to a user training system comprising at least a subset of any images identified by the image-based search.

The inventive realization according to embodiments of this invention is to realize that it is easier and more reliable to search an image database visually, using image-based searching, than it is to use more typical methods of searching using e.g. tagged metadata of the images. Image-based searching is a much more direct, reliable, robust way of searching for what is needed. Algorithms for image based searching already exist.

Therefore the inventive concept is to realize that the problem of obtaining image data in accordance with requirements can be solved by first converting linguistically represented criteria for the image (expressed for example via selection of options on an interactive UI) into a graphical representation, in the form of a constructed artificial base image, which is formed from a layered formation of different visual/graphical elements overlaid and size and position-adjusted.

The database can then be searched using the base image. The constructed base image itself would not be suitable for training purposes because it is an artificial construct, i.e. a rough visual seed used for searching (or for image generation using a neural network).

As will be explained below, in accordance with some embodiments, if the database finds no or few results, then a pre-trained AI model can be used to generate new images based on the pre-existing image.

In some embodiments, the received user inputs include at least: an input indicative of a desired anatomical structure, and an input indicative of a desired pathology associated with the anatomical structure. The selection of the plurality of pre-formed image elements may comprise selecting at least one image element representative of the anatomical structure and at least one image element representative of an anatomical feature associated with the pathology.

By way of non-limiting illustrative example, the anatomical feature associated with the pathology might be a tumor, a hemorrhage, a calcification, ischemia, or fracture.

In some embodiments, the received user inputs further comprise an indication of a desired imaging modality for the base image.

In some embodiments, the received user inputs further comprise an indication of a desired 2D/3D dimensionality of the image.

In some embodiments, the method comprises providing, via a display of the user interface device, a graphical user interface permitting input by the user of the said user inputs. Thus, this permits a user to efficiently transfer their linguistic understanding of the requirements for the image search into definable visual properties and features of the base image that will be used for the search.

In some embodiments, the graphical user interface comprises a set of input fields permitting input by the user of the desired anatomical structure and desired pathology.

In some embodiments, the method comprises constructing the base image in real time with receipt of each user input, and wherein the graphical user interface includes a preview pane showing the base image in a current form, and updating the base image in the preview pane following each further user input. Thus, the user can see the base image as it is built up, and this helps guide them in deciding any modifications that are needed, and in adding further image elements.

In some embodiments, the graphical user interface provides control functionality permitting drag-and-drop by a user of selected image elements relative to one another, for thereby indicating the relative positioning of the image elements relative to one another.

In some embodiments, the combining of the image elements to form the base images comprises overlaying at least one of the selected image elements atop at least one other of the selected image elements such that the base image has a layered formation.

In some embodiments, the method further comprises: responsive to determining that the number of images identified by the image-based search falls below a threshold number, accessing from a datastore a generative adversarial network (GAN) which is configured to receive as input the base image and to generate as output one or more simulated images. The method may comprise supplying the base image to the GAN to generate one or more simulated images. The method may comprise including the generated one or more simulated images in the data package for export.

In some embodiments, the method further comprises: comparing each of the one or more simulated images output from the GAN network against the base image; and presenting on the user interface an output indicative of a result of the comparison for each image. In some embodiments, the method further comprises receiving a user input from the user interface indicative, for each of the simulated images, of user acceptance of the image or user rejection of the image. In some embodiments, only images which are accepted by the user are included in said data package for export.

In some embodiments, in advance of performing the said comparison, an anatomical plausibility check is performed comprising processing each generated image output from the GAN with one or more algorithms, each algorithm configured to detect one or more features in the image and compare the features against one or more rules to determine an anatomical plausibility of the feature.

In some embodiments, the method further comprises receiving a training results report from the user training system, the results report indicative of a level of user training success in relation to one or more anatomical structures and/or pathologies of anatomical structures; determining based on the training results report one or more anatomical structures and/or pathologies of anatomical structures for which a level of user success is below a pre-defined threshold. The selection of the plurality of image elements from the set may be performed based on the said determined one or more anatomical structures and/or pathologies of anatomical structures.

Another aspect of the invention is a computer program product comprising computer program code configured when run on a processor to cause the processor to perform a method in accordance with any embodiments outlined in this document, or in accordance with any claim of this application.

Another aspect of the invention is a processing unit, comprising: an input/output; and one or more processors configured to perform a method. The method comprises: receiving at the input/output a set of user inputs from a user interface device; selecting a plurality of pre-formed image elements from a pre-defined set of pre-formed image elements, each image element being an image representation of an anatomical object or feature; defining a relative positioning and/or size of the selected one or more image elements, wherein the selection of the plurality of image elements from the set, and the defining of the positioning and/or size of the image elements is performed based on the user inputs received from the user interface device; constructing an artificial base image based on combining the selected image elements in accordance with the defined relative positioning and/or size(s); accessing an image database storing a dataset of medical images; performing an image-based searching operation comprising searching the image database based on the base image for finding images in the database similar to the base image; and generating a data package for export via the input/output to a user training system comprising at least a subset of any images identified by the image-based search.

In some embodiments, the received user inputs include at least: an input indicative of a desired anatomical structure, and an input indicative of a desired pathology associated with the anatomical structure. The selecting of the plurality of pre-formed image elements may comprise selecting at least one image element representative of the anatomical structure and at least one image element representative of an anatomical feature associated with the pathology.

In some embodiments, the method further comprises: responsive to determining that the number of images identified by the image-based search falls below a threshold number, accessing from a datastore a generative adversarial network (GAN) which is configured to receive as input the base image and to generate as output one or more simulated images; supplying the base image to the GAN to generate one or more simulated images; and including the generated one or more simulated images in the data package for export.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 is a block diagram of an example processing unit and system in accordance with one or more embodiments of the invention;
Fig. 3 illustrates examples of a set of pre-formed image elements for use in constructing a base image; and
Fig. 4 illustrates examples of different base images constructed by overlaying two or more pre-deformed image elements.
Fig. 5 illustrates an example process flow for determining requirements for generation of training material.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for retrieving relevant images from an image database based on converting linguistic search criteria into image-based search criteria. User inputs are used to construct a base image from a combination of pre-formed graphical or visual image elements, and an image database is then queried with the base image using image-based searching. The resulting retrieved images may then be exported to a user training system for use in training a user using the images.

In the context of training, access to a set of images relevant to a particular domain is of great value, since it allows training to be arbitrarily customized to trainee needs.

At present, training material sources typically comprise databases of material grouped or ordered based on the topic or domain. But in a case where an individual needs training in specific areas, the necessary training material sources might not be readily available or there may be a lack of material available. The process of searching for material is technically challenging due to the abundance of information. One main challenge lies in forming the right search query to either find relevant images in a database or to find the right image to be used as input seed to a neural network, such as a Generative Adversarial Network (GAN), for use in generating new images.

Embodiments of the present invention propose a new approach to overcome the problem of formulating the right search query. In some examples, this allows for curating a customized training material set for an individual based on their needs, and optionally also based on previous training results. The proposed method also has applications more broadly outside of the area of training; it can be used in any application area in which searching for images is needed or useful.

In accordance with at least one set of embodiments, the method is applied as part of an objective of curating training material (including images) for practice sessions of trainees in diagnostic analysis.

One or more particular embodiments of the invention may include one or more of the following advantageous features: generation of a base image based on user specifications using a user interface; a database with relevant information (including images); a GAN network to generate images similar to base image. Embodiments may include only a subset of these features.

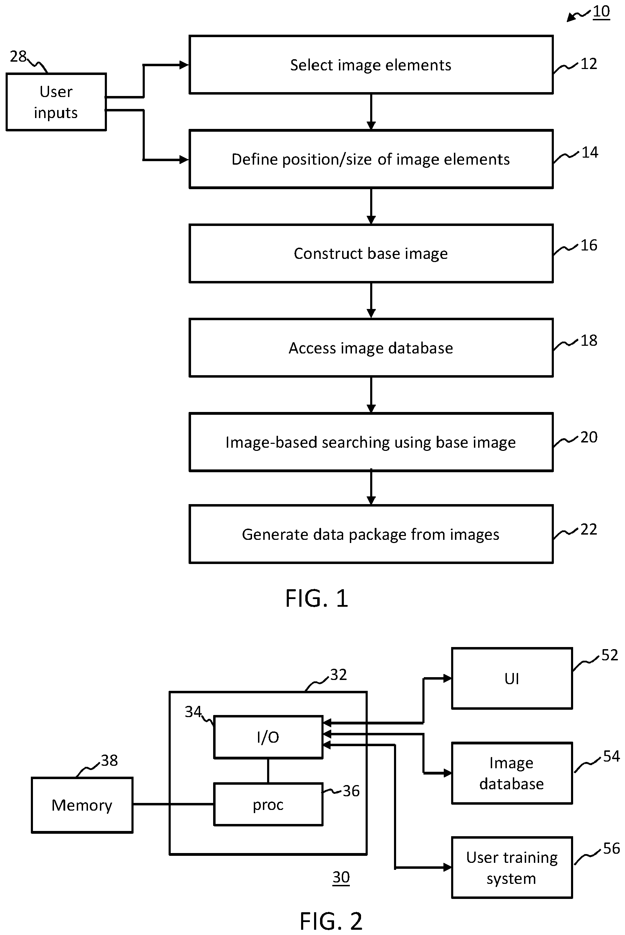

Fig. 1 outlines in block diagram form steps of an example computer implemented method 10 according to one or more embodiments. The steps will be recited in summary, before being explained further in the form of example embodiments.

The method 10 may be implemented by a processor or a computer.

The method comprises selecting 12 a plurality of pre-formed image elements from a pre-defined set of pre-formed image elements, each image element being an image representation of an anatomical object or feature.

The method further comprises defining 14 a relative positioning and/or size of the selected one or more image elements.

The selection of the plurality of image elements from the set, and the defining of the positioning and/or size of the image elements is performed based on user inputs 28 received from a user interface device. The method may comprise one or more steps of receiving the user inputs 28.

The method further comprises constructing 16 an artificial base image based on combining the selected image elements in accordance with the defined positioning and/or size.

The method further comprises accessing 18 an image database storing a dataset of medical images.

The method further comprises performing 20 an image-based searching operation comprising searching the image database based on the base image for finding images in the database similar to the base image.

The method further comprises generating 22 a data package for export to a user training system comprising at least a subset of any images identified by the image-based search.

As noted above, the method can also be embodied in hardware form, for example in the form of a processing unit which is configured to carry out a method in accordance with any example or embodiment described in this document, or in accordance with any claim of this application.

To further aid understanding, Fig. 2 presents a schematic representation of an example processing unit 32 configured to execute a method in accordance with one or more embodiments of the invention. The processing unit is shown in the context of a system 30 which comprises the processing unit. The processing unit alone represents an aspect of the invention. The system 30 is another aspect of the invention. The provided system does not have to comprise all of the illustrated hardware components; it may just comprise a subset of them.

The processing unit 32 comprises one or more processors 36 configured to perform a method in accordance with that outlined above, or in accordance with any embodiment described in this document or any claim of this application. In the illustrated example, the processing unit further comprises an input/output 34, or a communication interface.

The system 30 in this example further comprises a user interface 52.

The system 30 in this example further comprises an image database 54.

The system in this example further comprises a user training system 56.

In some embodiments, one or all of the user interface 52, image database 54 and user training system 56 could be components external to the system to which the processing unit 32 is configured to operatively couple during operation. Alternatively, these may be included in the provided system.

The invention may also be embodied in software form. Thus, an aspect of the invention is a computer program product comprising code means (computer program code) configured, when run on a processor, to cause the processor to perform a method in accordance with any of the embodiments described in this disclosure.

It is noted, with reference again to the system 30 of Fig. 2, that the system or the processing unit 32 may include a memory 38 which stores computer program instructions for execution by the one or more processors 36 of the processing unit to cause the one or more processors to perform a method in accordance with any of the embodiments described in this disclosure.

With regards to the "image elements" referred to above, these may each be individual images or image snippets which are designed to be combined to form the base image, e.g. by overlay of the various image elements on top of and/or adjacent to one another. For example, one image element might be a generic image of a slice through a brain. A second image element might be an image of a pathology within the brain. The second image element might be sized relative to the first image element so that it can be overlaid on the first image element to depict an anatomically accurate relative sizing of the pathology and the brain. For example, the pathology might be a hemorrhage or clot, a tumor a calcification, ischemia, fracture, or any other abnormality. Of course, these are just examples to illustrate the concept.

The reference to a "base image" simply means an image which is for use in a subsequent image-based searching operation, i.e. as an image for querying the image database 54. For example, it might otherwise be referred to as a 'query image'.

With reference to image-based searching, algorithms for performing such an operation will be known to the skilled person. Image-based searching simply means searching an image database using an image (a 'base image') as the query criterion or query item, for identifying images in the database similar (visually/graphically) to the base image. This type of searching is sometimes referred to as content-based image retrieval (CBIR). By way of example, image-based searching can employ use of techniques such as shape-matching, wavelet analysis, or AI-based comparison. For specific examples of techniques, reference is made to the following papers:
Rahman M.M., Bhattacharya P., Desai B.C. "A framework for medical image retrieval using machine learning and statistical similarity matching techniques with relevance feedback", IEEE Trans. Inf. Technol. Biomed., 11 (1) (2007), pp. 58-69;
Robinson G.P., et al. "Medical image collection indexing: shape-based retrieval using KD-trees", Comput. Med. Imaging Graph., 20 (4) (1996), pp. 209-217;
Orphanoudakis S.C., Chronaki C.E., Vamvaka D. "I2Cnet: content-based similarity search in geographically distributed repositories of medical images", Comput. Med. Imaging Graph., 20 (4) (1996), pp. 193-207;
El-Kwae E.A., Xu H., Kabuka M.R. Content-based retrieval in picture archiving and communication systems, J. Digit. Imaging, 13 (2) (2000), pp. 70-81.

With reference to the constructing of the base image, this comprises combining the selected image elements to form the base image, and with the relative sizing/positioning which is defined by the user inputs. In particular, the base image can be formed based on overlaying some of the image elements atop others to form a layered image structure. This is analogous for example to forming a collage from a plurality of image components, each separately formed, and which are positioned atop and/or adjacent to one another to construct a new fusion image. Thus, to state this as a more general principle, in some embodiments, the combining of the image elements to form the base image comprises overlaying at least one of the selected image elements atop at least one other of the selected image elements such that the base image has a layered formation.

In a preferred set of embodiments, the method may be implemented through use of a graphical user interface (GUI) 52 which the user can utilize in providing the user inputs 28 which guide the selection 12 of the image elements and the sizing and positioning of the image elements. The GUI may provide an interactive editing window in which the user can see the base image at each interim stage of its construction. For example, the graphical user interface may comprise a set of input fields permitting input by the user of relevant inputs. The method may comprise constructing the base image in real time with receipt of each user input, and wherein graphical user interface includes a preview pane showing the base image in a current form and updating the base image in the preview pane following each further user input. In some examples, the GUI may provide a control functionality permitting drag-and-drop by a user of selected image elements relative to one another, for thereby indicating the relative positioning of the image elements relative to one another.

Once the user has given the inputs to guide the construction of the base image, the image database 54 is queried using the constructed base image as a query image.

In an advantageous set of embodiments, the resulting set of images identified by the search are analyzed to determine whether they meet at least one criterion. For example, the number of images identified in the search may be counted and the at least one criterion may be a threshold number of images identified.

In some embodiments, if the resulting set of images fails to meet the at least one criterion, then a further phase of the method may be implemented comprising utilizing an artificial neural network to generate a set of one or more simulated images using the base image as a seed image. For example, a generative adversarial network (GAN) may be used for this purpose, where the GAN is configured to receive as input the base image and to generate as output one or more simulated images.

For example, in some embodiments, the method comprises, responsive to determining that the number of images identified by the image-based search falls below a threshold number, accessing from a datastore a generative adversarial network (GAN) which is configured to receive as input the base image and to generate as output one or more simulated images. The base image may be supplied as input to the GAN, and the GAN may generate one or more simulated images. At least a subset of the resulting generated one or more simulated images may be included in the previously reference data package for export.

In some embodiments, a further step could be implemented following the generation of the simulated images by the GAN. In particular, the one or more simulated images output by the GAN may be analyzed to determine whether they meet one or more criteria.

For example, the images generated by GAN might be compared with the base image and the delta variation derived from the comparison might be presented to the user or might be compared against a threshold. For example, this forms a feedback loop. If presented to the user on the UI 52, then the user may be provided by the UI the option to either accept the variation in the GAN-derived image or reject it. This might be performed for each image generated by the GAN. The accepted images are then stored for future use. Metadata may be created for the newly generated images and stored with the images to aid text-based retrieval in future based for example on the clinical scenario and domain.

Thus, to state this as a more general principle, in some embodiments, the method may comprise the following series of steps. The method may comprise comparing each of the one or more simulated images output from the GAN network against the base image. The method may further comprise presenting on the user interface 52 an output indicative of a result of the comparison for each image. The method may further comprise receiving a user input from the user interface indicative, for each of the simulated images, of user acceptance of the image or user rejection of the image. In some embodiments, the method may comprise that only images which are accepted by the user are included in said data package for export.

In some embodiments, additionally or alternatively to the above user-check, the method may comprise an automated analysis of the images output by the GAN. In some embodiments, this might include performing an anatomical plausibility check. In some embodiments such an anatomical plausibility check is performed by processing each generated image output from the GAN with one or more algorithms, wherein each of the one or more algorithms is configured to detect one or more features in the image and compare the features against one or more rules to determine an anatomical plausibility of the feature.

This step effectively provides a "sanity check" layer for the generated images. For example, the anatomical plausibility check may be a rule-based system which may evaluate whether the generated images are anatomically (and/or according to pathophysiology) plausible (e.g., in case of a tumor, whether there are compression effects on surrounding structures or whether blood accumulation traverses any unruptured membrane in case of a hemorrhage etc.). This might be implemented instead of or in addition to the above-described feedback loop with the user. If used in addition to the feedback loop, it may be implemented in advance of the feedback loop to reduce workload of the proctor/user.

For example, pathological lesions have well-documented behavior regarding how they grow and affect surrounding structures. One example for instance is that renal cell carcinomas often grow into the renal vein. Another example is that some malignancies erode surrounding capsules early in the history while others do not. Another example is that quickly growing brain lesions have different pressure effects than slow growing lesions. These represent illustrative examples. Such knowledge can be used to check the plausibility of images using suitable image analysis techniques.

In some embodiments, each image may be annotated with metadata which describes the anatomical location and pathology, e.g. an image of a lesion may already be annotated with the anatomical location and lesion type. Thus, in some embodiments, this metadata can be used as part of an anatomical plausibility check, e.g. to check whether the depicted pathology conforms to one or more pre-defined rules relating to the pathology. These rules may relate to expected relational characteristics with respect to other anatomical features in the image for example whether a pathology conforms to expected behavior. By way of illustrative example, a thrombosis/embolism image representing an hours to days old event should not be without infarction at the supplied region. By way of another example, in case of a tumor it is expected that there would be compression effects on surrounding structures. By way of another example, in the case of hemorrhage, it can be checked whether blood accumulation has traversed any unruptured membrane.

In terms of technical implementation, the anatomical plausibility check can involve image analysis techniques to detect presence of visual features which correspond to the pre-defined rules relating to the pathology. This can be based on well-known image analysis techniques such as shape-based matching or pattern matching, or use of a trained AI algorithm to detect the relevant features in the image associated with the pre-defined rules, to check whether the image meets the plausibility requirement.

As noted above, once the set of images is obtained, from the image-base search and/or from the GAN, then these may be incorporated in a data package for export. In some embodiments, these can be exported to a user training system for use as training material for training a clinician, for example in diagnostic analysis. In some embodiments, they may simply be exported to a datastore such as a memory or archive, which may be a physical storage medium, or may be cloud-based storage medium for example.

Further details will now be discussed regarding the generation of the base image. As discussed above, the selection of the image elements and of the relative positioning/size of the image elements is performed based on user inputs.

In some embodiments, the received user inputs may include at least: an input indicative of a desired anatomical structure, and an input indicative of a desired pathology associated with the anatomical structure, and wherein the selection of the plurality of pre-formed image elements comprises selecting at least one image element representative of the anatomical structure and at least one image element representative of an anatomical feature associated with the pathology. In other words, the user, with the user interface, indicates the desired anatomy and pathology which the images should include, and appropriate image elements are retrieved from a database of image elements based on those specified requirements. For example the anatomical feature associated with the pathology may be for example a tumor or a hemorrhage.

In some embodiments, the received user inputs may further comprise an indication of a desired imaging modality for the base image (e.g. MRI, CT, x-ray, ultrasound etc.).

In some embodiments, the received user inputs may further comprise an indication of a desired 2D/3D dimensionality of the base image.

An example implementation of the method according to a particular set of embodiments will now be described by way of illustration. It will be appreciated that not all features of this particular set of embodiments are essential to the inventive concept, and are described to aid understanding and to provide an example to illustrate the concepts.

The system in accordance with this set of embodiments includes an interactive user interface (UI) for use in generation of the base image. The workflow for forming the base image, may be as follows.

The user uses the UI to select a desired anatomy/organ (e.g.: brain, lung) to which the base image will relate. In some embodiments, this step might be aided by auto-suggestions (or auto-complete suggestions) populated using a natural language processing (NLP) engine, and wherein the auto-suggestions are presented on the graphical user interface and the user interface permits the user to select the options.

The user uses the UI to select a desired dimensionality of the base image - i.e. whether the base image should be 2D or 3D.

The user uses the UI to further select a clinical condition (pathology) associated with the anatomy/organ chosen in the preceding step (e.g. tumor, hemorrhage etc.). In some embodiments, the user may be presented with a list of automatically generated suggestions for possible pathologies based on the anatomy already selected in the previous step. In some embodiments, an NLP engine may be used which is configured to provide auto-suggestions for the pathology (e.g. auto-complete suggestions), which may be further filtered based on the anatomy chosen in the previous step (i.e. so that conditions specific to the anatomy are suggested).

The processing unit 32 may then use the user's inputs relating the desired anatomical structure and pathology to select an image element depicting the anatomical structure and an image element depicting an anatomical feature associated with the pathology.

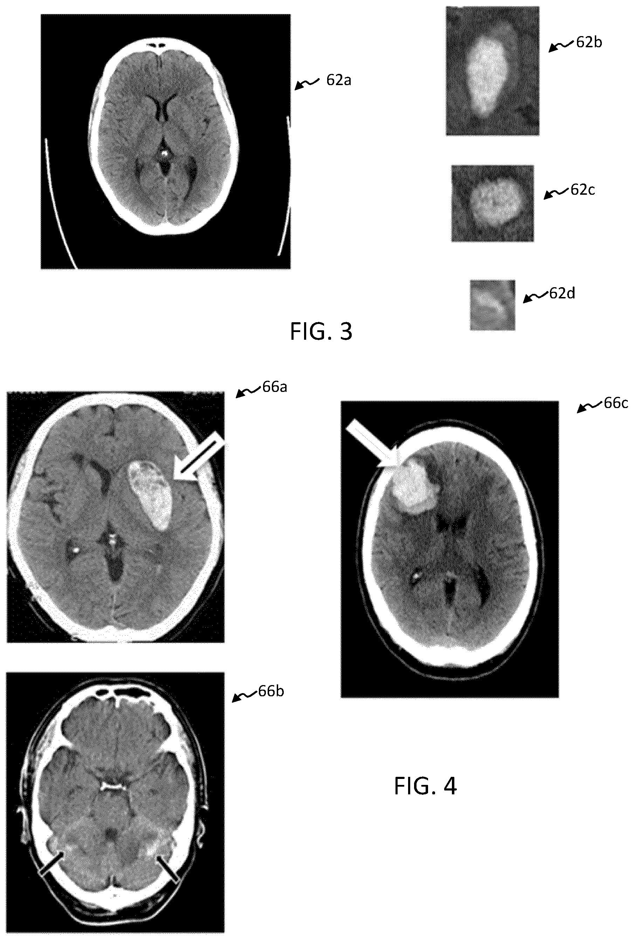

By way of example, Fig. 3 depicts example image elements 62. Element 62a is an example image element depicting an anatomical structure, the anatomical structure in this case being the brain. Elements 62b, 62c and 62d are image elements depicting anatomical features relating to a pathology of hemorrhage. Each is an image snippet depicting an example of a hemorrhage. The elements 62b, 62c and 62d differ in depicting different sizes and shapes of hemorrhage.

The user uses the UI to adjust a size of the image element corresponding to the pathology/condition (if applicable), e.g. one of 62b, 62c, 62d. For example, the user may adjust the size relative to the size of the image element (e.g. element 62a) corresponding to the anatomy. The size is configured by the user (which may be a trainer or trainee or any other type of user). For example, the graphical user interface may provide graphical control elements to permit the size adjustment.

The user may further select or configure the position of the image element corresponding to the pathology/condition relative to image element corresponding to the anatomy/organ (e.g.: anterior, posterior). Typically, the image element corresponding to the pathology will be overlaid atop the image element corresponding to the anatomy, and positioned within the borders of the anatomy depicted by the anatomy image element, i.e. inside it.

In some embodiments, the user may further select a desired imaging modality (and desired image sequence where appropriate). This might in fact be performed as an initial step in some embodiments.

The user may further be provided a control option to finalize and confirm the selections.

Based on the user's inputs via the user interface, a base image is generated based on the specifications, which can for example be further used to curate a training material set.

By way of illustration, Fig. 4 depicts examples of base images 66a, 66b, 66c which are composed of an image element corresponding to the anatomy of the brain and an overlaid image element corresponding to a hemorrhage, placed in the temporal lobe. In images 66a, 66c, the respective locations of the hemorrhage are indicated by the white arrows. In image 66b, there are two hemorrhage regions present, indicated by small black arrows at the bottom of the image. The image 66a is formed from a combination of image element 62a with image element 62b superposed atop. Image 66c is formed from a combination of image element 62a with image element 62c superposed atop. Image 66b is formed from a combination of image element 62a with two copies of image element 62d superposed atop to form two hemorrhage regions.

As noted above, in some embodiments, the base image can be constructed piecemeal, step-by-step, as the user inputs are provided in the user interface, and a preview pane on the user interface depicts the base image in its current state of formation at each stage in the construction process.

A searchable database (e.g. a database comprised by a PACS system) is then queried to find images similar to the base image.

The searching of the database employs image-based searching techniques well known in the art.

In some embodiments, a neural network (e.g. a GAN) is further provided for generating images similar to the base image, and might be uses e.g. only if the required number of images is not found through the search of the image database. This might occur for example most particularly where the anatomy or pathology is relatively less common, e.g. in case of rare clinical conditions.

In some embodiments, the UI may be used to generate a plurality of base images with the same pathology and relating to the same anatomy, but with minor variations, e.g. positional variations, size variations, intensity or resolution variations. This may form a base image set. Such a base image set can in some embodiments be used as input to a suitably trained GAN for generating output images which are similar to the set of images.

As has been discussed, embodiments of the invention are envisaged as having particularly advantageous application in forming training material for training clinicians in diagnostic image analysis.

In some embodiments, the formation of the base image may be informed in part by a received training results report from a user training system, the results report indicative of a level of user training success in relation to one or more anatomical structures and/or pathologies of anatomical structures. This can be used to automatically determine one or more anatomical structures and/or pathologies of anatomical structures for which a level of user success is below a pre-defined threshold. The selection of the plurality of image elements from the set may then be performed based on the said determined one or more anatomical structures and/or pathologies of anatomical structures. For example, both user-inputs and the results report can be used jointly to determine or select an appropriate set of image elements. In some embodiments, at least one base image might be generated based on both the results report and based on the user inputs to the user interface. In some embodiments, at least one base image might be generated based on the results report alone, and one base image based on the user inputs alone.

Another aspect of this invention is the provision of a training method for training a trainee in diagnostic analysis. This may be a method for remote training, i.e. a distant education scenario, where a proctor and trainee are involved.

Embodiments of the method described above may be employed to deliver additional training material, e.g. in scenarios where the individual requires special attention in one topic/domain. The training material may consist mainly of images. The images might be pre-annotated with metadata (e.g. with marked regions of interest and/or pixel level annotations) for the trainee to better understand a concept being represented by the image visually. The training material may mainly include images. The training system may include an interactive user interface which prompts a user to mark a particular region of interest or a particular pathology, or to identify a particular pathology. The user's inputs may be received at the user interface of the training system and compared against the ground truth answers stored as metadata of the image. Based on the comparison, feedback may be given to the trainee user, which thereby increases the scope for improvement.

Thus some embodiments of the invention may further comprise a training phase wherein, after export of the data package by the previously described processing unit 32 of the system 30, the user training system 56 receives the data package and implements a training program in relation to a trainee user by controlling a user interface of the user training system 56 to display one or more of the images included in the data package. In some embodiments, before starting such a training program, a trainer user may annotate one or more of the images in the exported data package with metadata which indicates ground truth 'solutions' which can be compared against trainee-input responses to the user interface during the training session responsive to each displayed image.

In some embodiments, and as described above, the results of a training session can be used to guide further generation of images using the image generation method 10 described previously.

For example, and by way of non-limiting illustration only, during a training session, a trainer/proctor shows an image, for example a CT brain image which depicts presence of a hemorrhage. The attendee(s) of the training session might not be able to correctly locate the hemorrhage with perfect accuracy, at least not with just one image. Once the training session is complete, a test image might be shared with all of the trainee attendees, and the trainees are prompted to mark the hemorrhagic region. The marked image is compared against the ground truth. Depending upon the accuracy of locating the hemorrhage, additional CT brain images might be generated using for example the method 10 described above, and/or simply using the previously discussed GAN(s). These images can be used for further practice.

In some embodiments, a level of satisfaction of the trainee can also be taken into account. The feedback might take place at every stage to inform when generation of additional images is needed and what type of images to generate, based on the user's test score results (indicating understanding level), and based on the user's own assessment of how happy they are with a particular topic (satisfaction score).

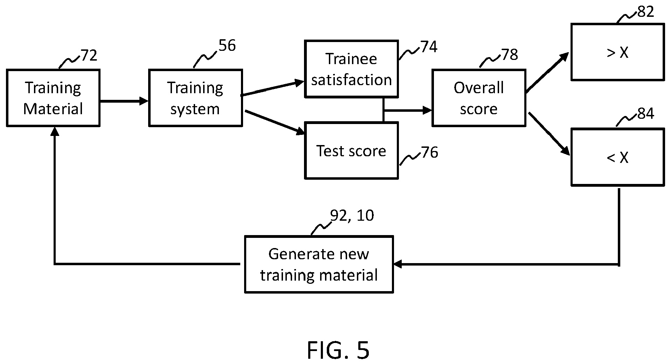

By way of illustration, Fig. 5 illustrates an example process flow for determining requirements for generation of training material.

The process comprises receiving a starting set of training material 72 including training images. The training images may be annotated with ground truth 'answers'. For example, this comprises receiving the data package from the processing unit 32 discussed previously. The training material is provided to a training system 56 which includes a user interface. The user interface is controlled to present to the user the training images and prompt the user for input relating to the image, e.g. prompting the user to identify a particular pathology by name or to spatially point to a pathology in the image using a pointer means, e.g. a mouse or touchscreen. The user answers are compared against the metadata answers tagged to the image to determine a test score 76. The user is also prompted to indicate how satisfied they are with their skill or performance to generate a trainee satisfaction score 74. The two scores are combined to form an overall score 78. The overall score is compared against a threshold, X. If the score is greater 82 than the threshold, then the training session might end. If the score is lower 84 than the threshold, then the training session may continue to a step 92 of generating new training materials which may include generating new training images, for example relating to the same anatomical area and/or the same pathology. This may employ use for example of the image generation method 10 already discussed earlier in this disclosure.

In some embodiments, the method for implementing the user training may include steps for further customizing the training session and/or training materials. For example, where the trainee user makes a mistake (e.g. their input answer to a prompt question does not match the correct answer stored as metadata to the test image), several strategies can be employed to build up the skill of the trainee.

In some embodiments, responsive to a user obtaining a low test score 76, the user may be presented with an 'easier' or more generic/textbook version of the image and then the difficulty of the images may gradually be increased to increase the complexity level. For this purpose, the metadata of each of the training images might include a tag indicative of a training difficulty level of the image, which might be set by a trainer user.

In some embodiments, images of higher difficulty may be presented in close temporal proximity with images of lower difficulty of the same anatomy and same pathology. This helps the user to resolve potential confusion which the more difficult images may cause. For example, in case of confusing lesions, images may be present in close temporal proximity as part of the training session/program that would resolve the confusion (e.g., image of the same lesion in another modality or another MRI sequence or from another view).

In some embodiments, the method may comprise receiving an input from the user relating to a user request for particular training images, e.g. an indication of a desired anatomy and pathology. The method may comprise performing analytics on said user input requests to resolve confusions.

Some embodiments described above make use of a generative adversarial network (GAN) in order to generate simulated images from the base image. Background information and implementation details for a GAN will now be briefly discussed.

Generative adversarial networks (GAN) are a type of machine learning model which seeks to identify a probability distribution underlying a provided training dataset, and then use this to generate new training data entries which can then be further used to refine the predictive network. Generative adversarial networks can be used to perform image-to-image translation where one input image (a seed image) is processed by the network to generate an output image. In the context of embodiments of the present invention, the input image is the base image, and one or more output images are images similar to the base image.

Within the field of machine learning, the most common type of machine learning is supervised machine learning. Here, a set of training data is constructed manually by a supervising human, who labels each training data entry with its corresponding ground truth, or the target output of the predictive model. The training of such models is limited due to the need for human input to annotate each training data entry. Generative networks are a type of machine learning which is non-supervised, and in particular which can computationally augment a starting training dataset to create a far greater set of training examples than would be possible in supervised learning. Image-to-image translation is an ideal application for GAN networks.

Reference is made for example to the paper Goodfellow I, et al. "Generative adversarial nets." In: Communications of the ACM, November 2020, Vol. 63 No. 11, Pages 139-144. This paper outlines in detail the principles behind the construction of a suitable Generative Adversarial Network. Section 3 in particular outlines the basic architecture of a Generative Adversarial Network, and also provides further references which expand on key points.

The paper Goodfellow, I, et al: "Generative adversarial nets." In Proceedings of the 27th International Conference on Neural Information Processing Systems - Volume 2 (NIPS'14). MIT Press, Cambridge, MA, USA, 2672-2680 provides a further explanation of the structure of Generative Adversarial Networks.

Reference is further made to the paper Alotaibi, A. "Deep Generative Adversarial Networks for Image-to-Image Translation: A Review." Symmetry 2020, 12(10), 1705. This paper provides a detailed overview of the use of GAN algorithms for image-to-image translation.

Two advantageous GAN architectures known for use in image-to-image translation are pix2pix and CycleGAN. The pixp2pix architecture is more appropriate for cases in which the available training data are paired, and CycleGAN is more appropriate for cases in which the available training data are non-paired. To explain further, the aim in the context of the present application is for the trained GAN-based machine learning model to take as input a base image, and to produce as output a variation on the base image which depicts the same anatomy (e.g. anatomical structure or region), and which depicts the same pathology, but which differs e.g. in positioning/size of the pathology or in certain characteristics of the anatomy.

For training a machine learning model to perform this image-to-image translation task for a certain set of scan parameters, the training data would comprise a plurality of example first images, and a plurality of example second images, the latter of which represent variations upon the first images of the type which it is desired that the GAN will produce in practice.

Paired training data in this context would mean that each second image had a corresponding one of the first images with which it was paired, meaning that it represented a particular variation upon the first image, e.g. depicting the same anatomy and pathology but different in the types of characteristics which it is desired the GAN would vary for forming the simulation images. Unpaired training data would mean that the first images and second images could simply be two sets of images of either the same anatomy and pathology or different anatomies and pathologies, but wherein the model is nonetheless able to identify patterns common to the first and second image sets, and generate a general network capable of mapping the one class of image data to the other. CycleGAN is able to handle unpaired training data.

Reference is made to the paper: P. Isola, J. et al, "Image-to-Image Translation with Conditional Adversarial Networks," 2017 IEEE Conference on Computer Vision and Pattern Recognition (CVPR), 2017, pp. 5967-5976. This paper describes the pix2pix architecture.

Reference is also made to the paper: J. Zhu, T. Park, P. Isola and A. A. Efros, "Unpaired Image-to-Image Translation Using Cycle-Consistent Adversarial Networks," 2017 IEEE International Conference on Computer Vision (ICCV), 2017, pp. 2242-2251. This paper describes the CycleGAN architecture.

These papers (Isola et al, and Zhu et al) provide full details of appropriate GAN-based machine leaning algorithms for application in embodiments of the present invention.

Deep learning networks can even be used to perform cross-modality image translation such as translating CT images to MRI images. Reference is made for example to Jin CB, Kim H, Liu M, et al. Deep CT to MR Synthesis Using Paired and Unpaired Data. Sensors (Basel). 2019;19(10):2361.

To generate the machine learning model(s), once the appropriate model architecture has been selected (e.g. pix2pix or CycleGAN described above), the model must be trained to perform the needed image-to-image translation between the base image and the output simulated images. For training, the training input images and training output images could both be real-world medical images of real patients, which are paired to relate to the same anatomy and pathology.

The GAN model may be anatomy-specific, i.e. trained for translating images of a particular anatomical region only. This might lead to slightly more accurate results. Alternatively, it could be non-anatomy-specific, capable of handling image data of any anatomical area.

Embodiments of the invention described above employ a processing unit. The processing unit may in general comprise a single processor or a plurality of processors. It may be located in a single containing device, structure or unit, or it may be distributed between a plurality of different devices, structures or units. Reference therefore to the processing unit being adapted or configured to perform a particular step or task may correspond to that step or task being performed by any one or more of a plurality of processing components, either alone or in combination. The skilled person will understand how such a distributed processing unit can be implemented. The processing unit includes a communication module or input/output for receiving data and outputting data to further components.

The one or more processors of the processing unit can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method comprising:
selecting a plurality of pre-formed image elements from a pre-defined set of pre-formed image elements, each image element being an image representation of an anatomical object or feature;
defining a relative positioning and/or size of the selected one or more image elements;
wherein the selection of the plurality of image elements from the set, and the defining of the positioning and/or size of the image elements is performed based on user inputs received from a user interface device;
constructing an artificial base image based on combining the selected image elements in accordance with the defined positioning and/or size,
accessing an image database storing a dataset of medical images;
performing an image-based searching operation comprising searching the image database based on the base image for finding images in the database similar to the base image;
generating a data package for export to a user training system comprising at least a subset of any images identified by the image-based search.

2. The method of claim 1, wherein the received user inputs include at least:
an input indicative of a desired anatomical structure, and
an input indicative of a desired pathology associated with the anatomical structure;
wherein the selection of the plurality of pre-formed image elements comprises selecting at least one image element representative of the anatomical structure and at least one image element representative of an anatomical feature associated with the pathology.

3. The method of claim 1 or 2, wherein the received user inputs further comprise an indication of a desired imaging modality for the base image.

4. The method of any of claims 1-3, wherein the received user inputs further comprise an indication of a desired 2D/3D dimensionality of the image.

5. The method of any of claims 1-4, wherein the method comprises providing via a display of the user interface device a graphical user interface permitting input by the user of the said user inputs.

6. The method of claim 5, wherein the graphical user interface comprises a set of input fields permitting input by the user of the desired anatomical structure and desired pathology.

7. The method of claim 5 or 6, wherein the method comprises constructing the base image in real time with receipt of each user input, and wherein graphical user interface includes a preview pane showing the base image in a current form, and updating the base image in the preview pane following each further user input.

8. The method of any of claims 5-7, wherein the graphical user interface provides control functionality permitting drag-and-drop by a user of selected image elements relative to one another, for thereby indicating the relative positioning of the image elements relative to one another.

9. The method of any of claims 1-8, wherein the combining of the image elements to form the base images comprises overlaying at least one of the selected image elements atop at least one other of the selected image elements such that the base image has a layered formation.

10. The method of any of claims 1-9, wherein the method further comprises:
responsive to determining that the number of images identified by the image-based search falls below a threshold number, accessing from a datastore a generative adversarial network (GAN) which is configured to receive as input the base image and to generate as output one or more simulated images;
supplying the base image to the GAN to generate one or more simulated images; and
including the generated one or more simulated images in the data package for export.

11. The method of claim 10, wherein the method further comprises
comparing each of the one or more simulated images output from the GAN network against the base image;
presenting on the user interface an output indicative of a result of the comparison for each image; and
receiving a user input from the user interface indicative, for each of the simulated images, of user acceptance of the image or user rejection of the image;
wherein only images which are accepted by the user are included in said data package for export.

12. The method of claim 11, wherein, in advance of performing the said comparison, an anatomical plausibility check is performed comprising processing each generated image output from the GAN with one or more algorithms, each algorithm configured to detect one or more features in the image and compare the features against one or more rules to determine an anatomical plausibility of the feature.

13. The method of any of claims 1-12, further comprising:
receiving a training results report from the user training system, the results report indicative of a level of user training success in relation to one or more anatomical structures and/or pathologies of anatomical structures;
determine based on the training results report one or more anatomical structures and/or pathologies of anatomical structures for which a level of user success is below a pre-defined threshold;
wherein the selection of the plurality of image elements from the set is performed based on the said determined one or more anatomical structures and/or pathologies of anatomical structures.

14. A computer program product comprising code means configured when run on a processor to cause the processor to perform a method in accordance with any of claims 1-13.

15. A processing unit, comprising:
an input/output; and
one or more processors configured to perform a method, wherein the method comprises:
receiving at the input/output a set of user inputs from a user interface device;
selecting a plurality of pre-formed image elements from a pre-defined set of pre-formed image elements, each image element being an image representation of an anatomical object or feature;
defining a relative positioning and/or size of the selected one or more image elements;
wherein the selection of the plurality of image elements from the set, and the defining of the positioning and/or size of the image elements is performed based on the user inputs received from the user interface device;
constructing an artificial base image based on combining the selected image elements in accordance with the defined relative positioning and/or size(s),
accessing an image database storing a dataset of medical images;
performing an image-based searching operation comprising searching the image database based on the base image for finding images in the database similar to the base image;
generating a data package for export via the input/output to a user training system comprising at least a subset of any images identified by the image-based search.
